# EUROPEAN PATENT SPECIFICATION

(11) **EP 1 804 822 B1**
(45) Date of publication and mention of the grant of the patent: **22.07.2009**
(21) Application number: 05794493.6
(22) Date of filing: 23.09.2005
(51) Int. Cl.: A61K 38/02, A61K 39/29, C07K 14/18

(54) **HCV VACCINES FOR CHRONIC HCV PATIENTS**
HCV IMPFSTOFF FÜR CHRONISCHE HCV PATIENTEN
VACCINS ANTI-VHC POUR PATIENTS ATTEINTS DU VHC CHRONIQUE

(30) Priority: 29.10.2004 EP 04450200
(43) Date of publication of application: 11.07.2007
(73) Proprietor: Intercell AG, 1030 Vienna (AT)
(72) Inventor: FRISCH, Jürgen, 35041 Marburg (DE); TAUBER, Erich, A-3424 Muchendorf a. d. Donau (AT); BÜRGER, Vera, A-1230 Vienna (AT); KLADE, Christoph, A-2700 Wr. Neustadt (AT); BUSCHLE, Michael c/o Glenmark Pharmaceuticals S.A., 2300 La Chaux-de-Fonds (CH); COHEN, Katherine, A-1070 Vienna (AT)
(74) Representative: Sonn & Partner Patentanwälte
(86) International application number: PCT/EP2005/054773
(87) International publication number: WO 2006/045677

(56) References cited:
- WO-A-01/81359
- WO-A-02/00687
- WO-A-97/30721
- WO-A-02/059148
- MANNS MICHAEL P ET AL: "Immunization with the therapeutic hepatitis C virus (HCV) peptide vaccine IC41 in 66 chronic hepatitis C non-responder patients" HEPATOLOGY, vol. 40, no. 4, Suppl. 1, October 2004 (2004-10), page 251A, XP009057865 & 55TH ANNUAL MEETING OF THE AMERICAN-ASSOCIATION-FOR-THE-STUDY-OF-LIVE R-DISEASES (AASLD); BOSTON, MA, USA; OCTOBER 29 -NOVEMBER 02, 2004 ISSN: 0270-9139

## Description

Hepatitis C Virus (HCV) is a member of the flaviviridiae chronically infecting about 170 million people worldwide. There are at least 6 HCV genotypes and more than 50 subtypes have been described. In America, Europe and Japan genotypes 1, 2 and 3 are most common. The geographic distribution of HCV genotypes varies greatly with genotype 1a being predominant in the USA and parts of Western Europe, whereas 1b predominates in Southern and Central Europe (Bellentani 2000). HCV is transmitted through the parenteral or percutan route, and replicates in hepatocytes. About 15% of patients experience acute self-limited hepatitis associated with viral clearance and recovery. About 80% of infected persons become chronic carriers. Infection often persists asymptomatically with slow progression for years, however ultimately HCV is a major cause of cirrhosis, end-stage liver disease and liver cancer (Liang 2000). Strength and quality of both HTL and CTL responses determine whether patients recover (spontaneously or as a consequence of therapy) or develop chronic infection (Liang 2000).

Standard therapy of HCV comprises a combination of pegylated interferon-alpha and the antiviral ribavirin (Fried 2002). Virologic responses (defined as the absence of detectable HCV RNA 24 weeks after cessation of therapy) are, depending on the genotype, however, achieved in only about 50% of HCV patients with the standard therapy. Moreover, there are many side effects associated with both interferon (IFN) and ribavirin. IFN (also in its pegylated form) stimulates the immune system in a non-specific manner, which causes substantival side effects including flu-like syndrome, fever, headache, arthralgia, myalgia, depression, weight loss, alopecia, leukopenia and thrombopenia⁶. These side effects can necessitate cessation of treatment for some subjects. Treatment with IFN is also contraindicated in subjects with pre-existing hematologic disease (leukopenia and thrombopenia due to liver cirrhosis with splenomegaly). Ribavirin can cause transient liver enzyme elevations and psychiatric symptoms, hemolysis and anemia and has been associated with myocardial infarction in subjects with coronary heart disease. Ribavirin also exhibits teratogenic, mutagenic and carcinogenic potency. Therefore, contraception is mandatory for fertile male and female subjects treated with ribavirin. Therefore, the low tolerability and the considerable side effects of this therapy clearly necessitate novel therapeutic intervention including therapeutic vaccines and new treatment options for chronic HCV infection are urgently required, due to the limitations and lack of effective treatments (Cornberg 2002).

The WO 01/81359 A1 discloses ribavirin derivatives for the treatment of chronic hepatitis C infections.

The problem underlying the present invention is to overcome the limitations of the standard interferon-alpha/ribavirin combination therapy by providing effective medicaments or pharmaceutical compositions especially a vaccine for the treatment of chronic HCV infections, especially for those patients who had not responded to or only partially responded to or had relapsed from primary standard HCV therapy.

Therefore the present invention provides the use of polycationic peptides for the preparation and manufacturing of medicaments or pharmaceutical compositions, comprising HCV antigens or HCV epitopes for the treatment of patients with HCV chronic infections. Preferably, the patients with HCV chronic infections are those who had not responded to, partially responded to or had relapsed from primary standard HCV therapy by a combination of pegylated interferon-alpha and the antiviral ribavirin. The medicaments or pharmaceutical compositions are preferably vaccines.

A patient is considered to have relapsed when HCV RNA becomes undetectable on the primary standard HCV therapy with Peg-interferon alpha and Ribavirin but is detected again after disclontinuation of the treatment. Persons in whom HCV RNA levels remain stable on treatment are considered non-responders, while those whose HCV RNA levels decline (e.g. by >2 logs), but never become undetectable, are referred to as partial responders.

The polycationic peptide(s) to be used according to the present invention may be any polycationic peptide, which shows the characteristic effects according to the WO 97/30721. Preferred polycationic peptides are selected from basic polyppetides, organic polycations, basic polyamino acids or mixtures thereof. These polyamino acids should have a chain length of at least 4 amino acid residues (WO 97/30721). Especially preferred are substances like polylysine, polyarginine and polypeptides containing more than 20 %, especially more than 50 % of basic amino acids in a range of more than 8, especially more than 20, amino acid residues or mixtures thereof. Other preferred polycations and their pharmaceutical compositions are described in WO 97/30721 (e.g. polyethyleneimine) and WO 99/38528. Preferably these polypeptides contain between 20 and 500 amino acid residues, especially between 30 and 200 residues.

These polycationic peptides may be produced chemically or recombinantly or may be derived from natural sources.

Cationic (poly)peptides may also be anti-microbial with properties as reviewed in {Ganz, T., 1999}. These (poly)peptides may be of prokaryotic or animal or plant origin or may be produced chemically or recombinantly (WO 02/13857). Peptides may also belong to the class of defensins (WO 02/13857). Sequences of such peptides can be, for example, found in the Antimicrobial Sequences Database under the following internet address:
http://www.bbcm.univ.trieste.it/∼tossi/pag2.html

Such host defence peptides or defensives are also a preferred form of the polycationic polymer according to the present invention. Generally, a compound allowing as an end product activation (or down-regulation) of the adaptive immune system, preferably mediated by APCs (including dendritic cells) is used as polycationic polymer.

Especially preferred for use as polycationic peptides in the present invention are cathelicidin derived antimicrobial peptides or derivatives thereof (International patent application WO 02/13857), especially antimicrobial peptides derived from mammalian cathelicidin, preferably from human, bovine or mouse.

Polycationic peptides derived from natural sources include HIV-REV or HIV-TAT (derived cationic peptides, antennapedia peptides, chitosan or other derivatives of chitin) or other peptides derived from these peptides or proteins by biochemical or recombinant production. Other preferred polycationic peptides are cathelin or related or derived substances from cathelin. For example, mouse cathelin is a peptide, which has the amino acid sequence NH₂-RLAGLLRKGGEKIGEKLKKIGQKIKNEFQKLVPQPE-COOH. Related or derived cathelin substances contain the whole or parts of the cathelin sequence with at least 15-20 amino acid residues. Derivations may include the substitution or modification of the natural amino acids by amino adds, which are not among the 20 standard amino acids. Moreover, further cationic residues may be introduced into such cathelin molecules. These cathelin molecules are preferred to be combined with the antigen. These cathelin molecules surprisingly have turned out to be also effective as an adjuvant for an antigen without the addition of further adjuvants. It is therefore possible to use such cathelin molecules as evident adjuvants in vaccine formulations with or without further immunactivating substances.

Another preferred polycationic peptide to be used according to the present invention is a synthetic peptide containing at least 2 KLK-motifs separated by a linker of 3 to 7 hydrophobic amino acids (International patent application WO 02/32451. In the WO 02/32451 a type 1 inducing adjuvant (Immunizer) that is able to strongly enhance the immune response to a specific co-administered antigen and therefore constitutes a highly effective adjuvant is disclosed. The adjuvant (Immunizer) according to the WO 02/32451 is a peptide comprising a sequence R1-XZXZNXZX-R2, whereby N is a whole number between 3 and 7, preferably 5, X is a positively charged natural and/or non-natural amino acid residue, Z is an amino acid residue selected from the group consisting of L, V, I, F and/or W, and R1 and R2 are selected independantly one from the other from the group consisting of -H, -NH2, -COCH3, -COH, a peptide with up to 20 amino acid residues or a peptide reactive group or a peptide linker with or without a peptide; X-R2 may also be an amide, ester or thioester of the C-terminal amino acid residue. A specifically preferred peptide is KLKLLLLLKLK.

According to the present invention, the polycationic peptides can be used for the manufacturing of any medicaments, pharmaceutical compositions, especially vaccines which can be used for the treatment of chronic HCV infection, particularly for the treatment of those patients who had not responded to, partially responded to or had relapsed from primary standard HCV therapy by a combination of pegylated interferon-alpha and the antiviral ribavirin.

Preferably, the polycationic peptides are used for the manufacturing of medicaments, pharmaceutical compositions, especially vaccines that comprise HCV antigens, HCV hotspot epitopes and HCV epitopes. Most preferably, the HCV epitopes that are described in WO 01/24822 and PCT/EP2004/007540. Specifically preferred HCV epitopes therefore include: one or more, especially two, three, four, five or six of the following epitopes: MWNFISGIQYLAGLSTLPGN, HMWNHSGI, NHSGIQYLAGLSTLPGNPA, HMWNFISGIQYLAGLSTLPGNPA, IGLGKVLVDILAGYGAGVAGALVAFK AAWYELTPAETTVRLR, DYPYRLWHYPCTVNYTIFKI, DYPYRLWHYPCTVNFTIFKI, AYSQQTRGLL, TAYSQQTRGLLG, SMSYTWTGALITP, SPGALWGVI, LPRRGPRL, IGLGKVLVDILAGYgAGVAGALVAFK, GSIGLGKVLVDILAG, IGLGKVLVDILAGYG, LGKVLVDILAGYGAG, LVDILAGYGAGVAGA, DILAGYGAGVAGALV, LAGYGAGVAGALVAF, AAWYELTPAETTVRLR, AGAAWYELTPAETTV, AAWYELTPAETTVRL, TAYSQQTRGLLG, TAYSQQTRGLLGCTV, SMSYIWTGALITP, VVCCSMSYTWTGALITPC, VDYPYRLWHYPCTVNFTIFKVRMYVGGVEHRL, DYIPYRLWHYPCTVNF, YPYRLWHYPCTVNFT, LWHYPCTVNFTIFKV, TVNFTIFKVRMYVGG, TIFKVRMYVGGVEHR, VDYPYRLWHYPCTVNYTIFKIRMYVGGVEHRL, DYPYRLWHYPCTVNYTIFKI, DYPYRLWHYPCTVNY, LWHYPCTVNYTIFKI, TVNYTIFKTRMYVGG, TIFKIRMYVGGVEHR, RMYVGGVEHRL, HMWNFISGIQULAGLSTLPGNPA, NFISGIQYLAGLSTLPGNPA, MWNFISGIQYLAGLSTLPGN, KFPGGGQIVGGVYLLPRRGPRLGVRATRK, KFPGGGQIVGGVYLLPRRL, YLLPRRGPRL, LPRRGPRL, GPRLGVRAT, RLGVRATRK, GYKVLVLNPSVAAT, AYAAQGYKVL, AYAAQGYKVLVLNPSVAAT, DLMGYIPAV, GYIPLVGAPL, DLMGYIPLVGAPL, CINGVCWTV, GEVQWSTATQSFLAT, GEVQVVSTATQSFLATCINGVCWTV, HMWNHSCIQYLAGLSTLPGNPA, MWNFISGIQYLAGLSTLPGN, NFISGIQYLAGLSTLPGNPA, QYLAGLSTL, HMWNFISGI, VDYPYRLWHYPCTVNFTIFKVRMYVGGVEHRL, DYPYRLWHYPCTVNFTIFKI, DYPYRLWHYPCTVNFTIFKV, VDYPYRLWHYPCTVNYTIFKIRMYVGGVEHRL, DYPYRLWHYPCTVNYTIFKI, DYPYRLWHY, TVNYTIFKI, TINYTIFK, TVNFTIFKV, HYPCTVNYTI, HYPCTVNFTI, RMYVGGVEHR, AAWYELTPAETTVRLR, TPAIETIVRL, GWRLLAPITAYSQQTRGLLGCIV, TAYSQQTRGLLGCIV, TAYSQQTRGLLG, GQGWRLLAPITAYSQ, RLLAPITAY, GQGWRLLAPITAYSQQTRGLLGCIV, GQGWRLLAPITAYSQQTRGLLG, AYSQQTRGLL, AYSQQTRGL, IGLGKVLVDILAGYGAGVAGALVAFK, ILAGYGAGV, VAGALVAFK, GYGAGVAGAL, WCCSMSYTWTGALITPC, SMSYTWTGALITP, SMSYTWTGAL, SYTWTGALI, FTDNSSPPAVPQTFQV, LEDRDRSELSPLLLSTTEW, LEDRDRSELSPLLLST, RSELSPLLL, ELSPLLLST, DRDRSELSPL, LEDRDRSEL, LEDRDRSEL, YLVAYQATVCARAQAPPPSWD, YLVAYQATV, MSTNPKPQRKTKRNTNR, PQRKTKRNTNR, QRKTKRNTN, RKTKRNTNR, MSTNPKPQR, MSTNPKPQK, LINTNGSWHINRTALNCNDSL, NGSWHINRTALNCNDSL,LINTNGSWHI, RTALNCNDSL, LINTNGSWHIRTALN, SWHINRTALN, TTILGIGTVLDQAET, TTILGIGTV, TILGIGTVL, FDSSVLCECYDAGAAWYE, FDSSVLCECYDAGCA, VLCECYDAGA, VVLCECYDAGAAWYE, ARLIVFPDLGVRVCEKMALY, ARLIVFPDL, RLIVFPDLGV, RVCEKMALY, AFCSAMYVGDLCGSV, GVLFGLAYFSMVGNW, TRVPYFVRAQGLIRA, TTLLFNILGGWVAAQ LLFNILGGWV or fragments containing the T-cell epitope (at least 7, preferably at least 8, even more preferred at least 9 amino acid residues long). Preferably, the vaccine according to the present invention contains two or more, even more preferred three or more, especially four or more of such epitopes in combination. Preferred vaccines contain 3, 4, 5, 6 or 7 individual epitopes in one preparation (or two preparations stored and reconstituted seperately and applied together).

The medicament used according to the present invention is preferably used for induction of CD4+ Helper-T-cells and CD8+ cytotoxic T-cells. A specifically preferred field of use is the application of the medicament to a special group of patients: the use of the present medicament for replacing or supplementing a HCV standard therapy with interferon alpha and ribavirin, especially in patients where such standard therapy is not effective or not effective anymore.

Preferably, the medicament according to the present invention is used in combination with standard treatment such as interferon treatment. Preferably, it can be used for inducing type I T-cell responses in chronic HCV patients, and/or for inducing similar T-cell responses as seen during/after successful standard therapy, and/or for increasing responder rates and/or reducing relapse rates after standard therapy. In particular, the medicament according to the present invention is used in clinical trials as late add-on to standard therapy; the results confirm the excellent safety of the medicament, which does not exacerbate the side-effects of standard therapy such as leukopenia.

Preferably the medicament according to the present invention is specifically designed for HCV genotype 1. Genotype 1 patients have shown the lowest responder rates during standard therapy. Thus due to its ability to induce type I T-cell responses in chronic HCV patients, the medicament according to the present invention is preferably used to replace or supplement ribavirin (i.e. IFN/IC41 instead IFN/riba).

The medicament according to the present invention can be also used in combination with small-molecule protease inhibitors. Preferably, it can be used for inducing type I T-cell responses in chronic HCV patients and/or for inducing similar T-cell responses as seen during/after successful standard therapy. The medicament according to the present invention hence can offer a mode-of-action distinctly different from small-molecule inhibitors. It can hence complement efficacy of small molecule inhibitors in terms of response rates, duration of response, relapse rates, optimal dose/schedule. The medicament according to the present invention has no significant side effects, can in particular improve the efficacy/side-effect ratio of a small molecule inhibitor.

The medicament according to the present invention can be used in combination with imiquimod (a TLR7 agonist in the already licensed product Aldara/3M). Especially the local mobilization of antigen-presenting cells can increase the immunological potency of the medicament according to the present invention. In particular, stronger responses against more peptides after less vaccinations can be expected; the immunological responder rates and duration of responses and hence also virological responses can be increased; the stronger T-cell responses against more peptides can overcome the RNA rebound effect e.g. as seen in the IC41-201 study using the medicament according to the present invention.

The present invention also encompasses a method of treating the HCV patients described herein with an effective amount of the described medicament.

The present invention is further illustrated by the following figures, examples from which further features, embodiments and advantages may be taken. It is to be understood that the present examples are given by way of illustration only and not by way of limitation of the disclosure.

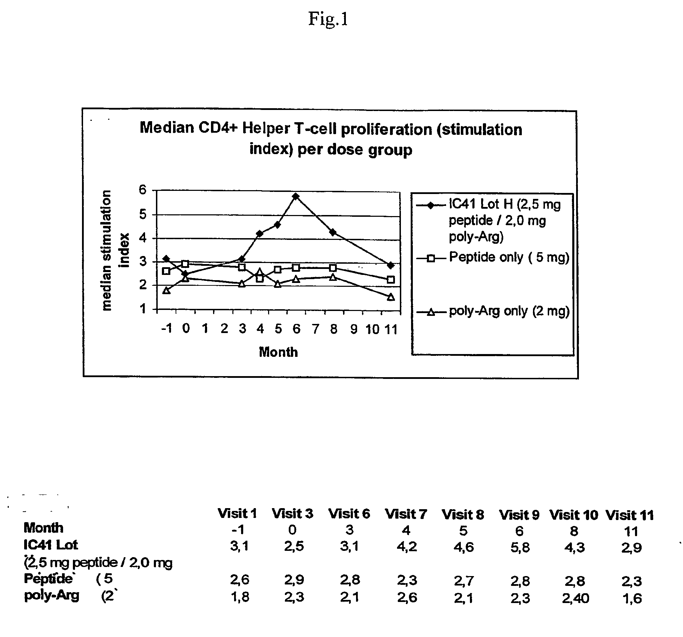

**Figure 1****:** CD4+ Helper-T-cell proliferation in chronic HCV patients. IC41 but not peptides alone, or poly-L-Arginine alone can induce significant proliferation. Vaccinations were given at month 0, 1, 2, 3, 4, 5. Stimulation indices for the IC41 peptides capable of inducing CD4+ Helper-T-cell responses were individually determined, significant responses were added up. Median responses of all 12 patients per dose group are shown.

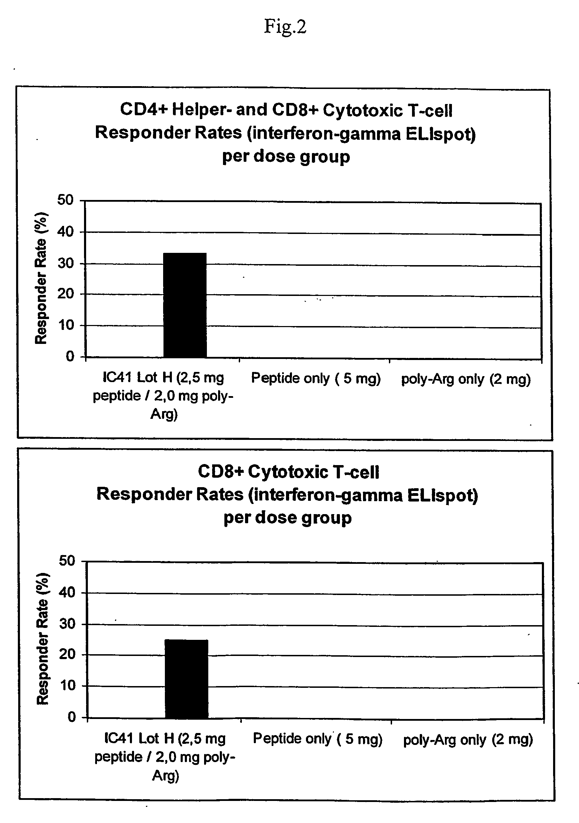

**Figure 2****:** Interferon-gamma ELIspot Responder Rates of patients vaccinated with IC41 or peptide alone or poly-L-Arginine alone. Each group consisted of 12 patients. Top panel: CD4+ Helper- and CD8+ cytotoxic T-cells, bottom panel: CD8+ cytotoxic T-cell, only.

### EXAMPLES:

### Example I: Human clinical study of HCV peptide vaccine in chronic HCV patients:

The first vaccine, where Poly-L-Arginine has been applied in humans is a fully synthetic therapeutic Hepatitis C Virus (HCV) vaccine. This vaccine was named IC41 and consists of a mixture of synthetic peptides representing conserved T cell epitopes of HCV plus Poly-L-Arginine as a synthetic T cell adjuvant. IC 41 comprises five peptides from different regions from the HCV polypeptide, i.a. the following three epitopes: HMWNFISGIQYLAGLSTLPGNPA, CINGVCWTV and DLMGYIPAV. The aim of this therapeutic approach is to restore a so-called type I T cell response against HCV in chronically infected patients. Such a response is typically seen in the around 15% of infected persons who do not proceed to chronicity but can clear HCV during the acute phase of infection. Since the pre-clinical experience with Poly-L-Arginine described earlier has shown its ability to induce type I immune responses in animal models it represents a promising T cell adjuvant for peptide vaccines for the treatment of HCV.

Various doses of the mentioned vaccine have already been tested in several clinical trials comprising more than 200 subjects: in an initial phase 1 study, safety and preliminary immunogenicity data of several doses were obtained. Results from that trial prompted initiation of a dose optimization study comprising 128 healthy volunteers in 10 different dose groups. The study was a randomized, single blind, parallel group, controlled study conducted to assess dose optimization and safety of the HCV peptide vaccine, IC41, in healthy subjects and was conducted in one center in Austria. One-hundred and twenty-eight subjects were randomly assigned to receive one of seven different doses and ratios of HCV peptide vaccine with Poly-L-Arginine, HCV peptide vaccine alone, Poly-L-Arginine alone or saline solution. All subjects received four administered vaccinations in monthly intervals. Immunogenicity was assessed at each of these time points at one month respectively and three months after the last vaccination.

The T cell stimulatory efficacy of Poly-L-Arginine was tested in a phase 2 clinical trial in chronic Hepatitis C Virus patients, who did not respond to or relapsed from standard interferon/ribavirin therapy.

For the present invention, a randomized, double blind study of HCV peptide vaccine, IC41, was conducted in patients with chronic HCV who had not responded to or had relapsed from primary standard HCV therapy. The study was conducted in 11 centers in Germany, Austria and Poland. Sixty patients were randomly assigned to receive three different doses and rations of HCV peptide vaccine with Poly-L-Arginine, HCV peptide vaccine alone or Poly-L-Arginine alone.

Male and female patients who met the following inclusion criteria were included in the study:
- Diagnosis of chronic hepatitis C, with a documented course of at least six months (ALT > 1.5 times the upper limit of normal [ULN] at two or more timepoints).
- Non-response to or relapse from primary standard HCV therapy of six to 12 months (depending on the genotype of HCV).
- HCV-RNA positive.
- HLA-A2 positive.
- HCV antibodies positive.
- Liver biopsy within 30 months prior to inclusion, demonstrating hepatic inflammation and/or fibrosis.
- Hematology and biochemistry laboratory results within the limits normally expected for the patient population (liver values maximum of 5x ULN).
- Aged 18 to 65 years.
- Written informed consent obtained prior to study entry.

In the present clinical study, each group consisted of 12 subjects, positive for HLA-A2. Subjects received 6 vaccinations in monthly intervals (at visits 3 to 8). Blood for immunological analyses was drawn prior vaccination and at visits 6 to 8, one month after last vaccination (visit 9), 3 months after last vaccination (visit 10) and 6 months after last vaccination (visit 11). For immunological monitoring of clinical trials, state-of-the-art T cell assays to determine immunological endpoints under GLP/GCP compliance were applied: Interferon-gamma ELIspot Assay, T cell Proliferation Assay, HLA-tetramer/FACS assay. These assays allow reliable measurements of epitope-specific T cell responses induced by the therapeutic HCV vaccine IC41. The vaccine-induced T cell immune responses serve as surrogate parameters of efficacy (Keilholz et al. 2002).

As primary endpoint T-cell immunogenicity was determined by a T-cell proliferation assay. The proliferation assay allows detection of peptide-specific T cells in biological samples like human blood. The basis of the assay is that, T cells upon stimulation with a peptide specifically recognized by their T cell receptor, react by secretion of cytokines and subsequent proliferation. Proliferation of cells can be measured by a variety of means; among the most sensitive approaches ranks incorporation of radioactively labeled thymidine into DNA synthesized prior cell division. This reaction can be carried out in a 96-well plate. Each well contains a fixed number of cells, which are cultured in the presence of antigen/peptide for a couple of days. For the last 16-20 hours thymidine labelled with tritium (3H-thymidine) is added. Cells are then harvested onto a filter plate: medium containing free radioactivity is washed away, whereas DNA sticks to the filter. Incorporated radioactivity can be quantified by means of a beta-scintillation counter determining counts-per-minute (cpm). The usual output is given as stimulation index (S.I.), which is defined as cpm of the test sample divided through cpm of the negative control.

As secondary endpoints T-cell immunogenicity was determined by interferon gamma ELIspot. ELIspot allows quantification of peptide-specific, functional (i.e. cytokine- secreting) T cells in biological samples like human blood. The basis of the assay is that, T cells upon stimulation with a peptide specifically recognized by the T cell receptor react by secretion of cytokines like IFN-γ. This reaction can be carried out in a 96-well plate. The filter-wells of this plate are coated with a Mab specific for IFN-γ. Consequently, each cell secreting IFN-γ leaves an IFN-γ spot, which can be visualized with a subsequent color reaction. Spots can be counted using automated plate readers. Numbers obtained are a measure for the frequency of peptide-specific, IFN-γ-secreting T cells in the sample.

As additional secondary endpoint HLA-tetramer/FACS analysis was performed. HLA class I tetramers, soluble recombinant forms of a complex of HLA molecule and antigenic peptide, bind the antigen-specific T cell receptor used for T cell recognition. By using flow cytometry with fluorescent tetramers, antigen-specific CD8⁺ T lymphocytes can be reliably enumerated and characterized. The assay uses HLA-A*0201 custom-made iTag™-tetramers produced by Beckman Coulter Immunomics complexed with IC41 class I epitopes.

Subjects were classified as responders if they showed significant T-cell responses at any of visits 4 to 11 and had no response prior treatment. In the case of pre-existing immunity (significant T-cell response against any peptide within IC41 already prior vaccination), an increase of at least 3 times of the pre-existing value was required to classify the effect as response.

As a first important result, these clinical trials confirmed the excellent safety profile of completely synthetic peptides in general and Poly-L-Arginine in particular. Furthermore, several important lessons regarding activation of human T cells were learned: in both studies, T cell responses were assessed using [³H]-thymidine proliferation and IFN-γ ELIspot assays, and flow cytometry (FACS). These assays, which have been standardized and validated at Intercell AG's Clinical Immunology Laboratory enable reliable measurements of epitope-specific T cell responses induced by vaccination. All assays were performed in compliance with Good Laboratory Practice (GLP)/Good Clinical Practice (GCP) requirements. Standardization of the blood cell isolation procedure at the different investigational sites led to a high rate of evaluable assays. However, due to the lack of inter-laboratory standardization of T cell assays, comparison of the results of this study with published data from similar trials is difficult. Cryo-preserved blood cells were used, resulting in possible underestimation of T cell responses compared with assays that utilize fresh blood.

In the phase 2 study population of chronic HCV patients a slightly different picture was obtained: in general, CD4+ and CD8+ T cell responses to IC41 peptides were more frequent and more vigorous in peripheral blood samples from those patients who were immunized with peptide and Poly-L-Arginine together, than in samples from those patients who were immunized with peptide or Poly-L-Arginine alone, confirming the requirement of Poly-L-Arginine as a T cell adjuvant. Vaccine responder rates were approximately two to three-fold higher in the verum groups than in the control groups (Fig. 1). T cell proliferation responders were more numerous in the verum groups (30-60%) than in the control groups (0-17%).

Most importantly however, interferon-gamma ELIspot responders were observed exclusively in the verum groups (Fig. 2). These results demonstrated for the first time that Poly-L-Arginine is able to induce type I responses even in the setting of chronic HCV infection in patients who could not be cured by the interferon/ribavirin standard therapy. Significant Interferon-gamma ELIspot responses were detected against both HLA-class II (recognized by helper T cells) and HLA-class I (recognized by cytotoxic T cells) peptides. Analysis of data up to and including those obtained at the second immunological check (Visit 11) performed 6 months after the last immunization revealed no important differences in immunological responder rates compared with the data obtained at Visits 1 to 10, indicating sustainability of the IC41-induced immune response. The study also disclosed that T cell immunity against the virus can be raised to a level that is not too different from the one induced in healthy vaccines. Thus, immunosuppression may not be as prevalent as anticipated in patients. It remains to be elucidated, how such T cell responses can be optimally applied to reduce disease progression or ameliorate symptoms and eventually clear the infection.

Taken together, Poly-L-Arginine represents one of the first synthetic T cell adjuvants, which has consistently - from in vitro experiments up to incurable chronically infected patients - been able to induce and augment the desired kind of immune response. Its easy manufacturability, excellent safety profile and its efficacy even in such difficult settings as chronic HCV infection, make it a promising new tool in the fight against infectious diseases and cancer.

### Example II: CD4+ Helper-T-cell proliferation in chronic HCV patients can be induced by IC41, but not by peptides alone, or poly-L-Arginine alone

As shown in Figure 1, IC41 is able of inducing a significant proliferative response in chronic HCV patients. Interestingly, neither peptides alone nor poly-L-Arginine alone have this ability, proofing the adjuvant effect of poly-L-Arginine.

### Example III: poly-L-Arginine is required to induce type I (interferon gamma) T-cell responses in chronic HCV patients

As shown in Figure 2, IC41 can induce interferon gamma secreting T-cells in chronic HCV patients, whereas peptides alone or poly-L-Arginine alone cannot induce any response. Importantly, both CD4+ Helper-T-cells and CD8+ cytotoxic T-cells can be induced.

Interferon gamma secretion is a hallmark of type I T-cell responses. Such responses are seen during the acute phase of infection in the subset of HCV patients, who eliminate the virus and do not proceed to chronic infection. Type I T-cell responses are also seen in patients undergoing standard therapy with interferon and ribavirin. Thus, induction of type I T-cell responses as achieved by IC41 is a primary goal of therapeutic vaccination against HCV.

References

Bellentani S, Miglioli L, Masutti F, Saccoccio G, Tiribelli C. Epidemiology of hepatitis C virus infection in Italy: the slowly unraveling mystery. Microbes Infect. 2000 Nov;2(14):1757-63.
Liang TJ, Rehermann B, Seeff LB, Hoofnagle JH. Pathogenesis, natural history, treatment, and prevention of hepatitis C. Ann Intern Med. 2000 Feb 15;132(4):296-305.
Cornberg M, Wedemeyer H, Manns MP. Treatment of chronic hepatitis C with PEGylated interferon and ribavirin. Curr Gastroenterol Rep. 2002 Feb;4(1):23-30.
Keilholz U, Weber J, Finke JH, Gabrilovich DI, Kast WM, Disis ML, Kirkwood JM, Scheibenbogen C, Schlom J, Maino VC, Lyerly HK, Lee PP, Storkus W, Marincola F, Worobec A, Atkins MB. Immunologic monitoring of cancer vaccine therapy: results of a workshop sponsored by the Society for Biological Therapy. J Immunother. 2002 Mar-Apr;25(2):97-138. Review.
Fried M, Shiffman M, et al. Peginterferon Alfa-2a plus ribavirin for chronic hepatitis C virus infection. N Engl J Med, Vol. 347, No. 13. September 26, 2002; 975-982.

## Claims

1. Use of polycationic peptides for the preparation and manufacturing of medicaments or pharmaceutical compositions, comprising HCV antigens or HCV epitopes for the treatment of patients with HCV chronic infections.

2. The use according to claim 1, wherein the medicaments or pharmaceutical compositions are vaccines.

3. The use according to claim 1 or 2, **characterised in that** the patients are those who had not responded to, partially responded to or had relapsed from primary standard HCV therapy by a combination of pegylated interferon-alpha and the antiviral rib-' avirin.

4. The use according to any one of the claims 1 to 3, **characterised in that** the polycationic peptides contain between 20 and 500 amino acid residues.

5. The use according to claim 4, **characterised in that** the polycationic peptides contain between 30 and 200 amino acid residues.

6. The use according to any one of claims 1 to 5, **characterised in that** the polycationic peptide is polyarginine or polylysine.

7. The use according to any one of claims 1 to 6, **characterised in that** the medicament is used for induction of CD4+ Helper-T-cells and CD8+ cytotoxic T-cells.

8. The use according to any one of claims 1 to 7, **characterised in that** the medicament is used for replacing or supplementing a HCV standard therapy with interferon alpha and ribavirin.

9. The use according to claim 8, **characterized in that** the medicament is for patients where such standard therapy is not effective or not effective anymore.

## Patentansprüche

1. Verwendung von polykationischen Peptiden zur Zubereitung und Herstellung von Medikamenten oder pharmazeutischen Zusammensetzungen, umfassend HCV-Antigene oder HCV-Epitope zur Behandlung von Patienten mit chronischen HCV-Infektionen.

2. Verwendung nach Anspruch 1, wobei die Medikamente oder die pharmazeutischen Zusammensetzungen Vakzine sind.

3. Verwendung nach Anspruch 1 oder 2, **dadurch gekennzeichnet, dass** die Patienten jene sind, die auf primäre HCV-Standardtherapie durch eine Kombination von PEGyliertem Interferon-Alpha und dem antiviralen Ribavirin nicht oder teilweise angesprochen oder einen Rückfall erlitten haben.

4. Verwendung nach einem der Ansprüche 1 bis 3, **dadurch gekennzeichnet, dass** die polykationischen Peptide zwischen 20 und 500 Aminosäurereste enthalten.

5. Verwendung nach Anspruch 4, **dadurch gekennzeichnet, dass** die polykationischen Peptide zwischen 30 und 200 Aminosäurereste enthalten.

6. Verwendung nach einem der Ansprüche 1 bis 5, **dadurch gekennzeichnet, dass** das polykationische Peptid Polyarginin oder Polylysin ist.

7. Verwendung nach einem der Ansprüche 1 bis 6, **dadurch gekennzeichnet, dass** das Medikament zur Induktion von CD4+-Helfer-T-Zellen und CD8+-cytotoxischen T-Zellen verwendet wird.

8. Verwendung nach einem der Ansprüche 1 bis 7, **dadurch gekennzeichnet, dass** das Medikament zum Ersetzen oder Ergänzen einer HCV-Standardtherapie mit Interferon-Alpha und Ribavirin verwendet wird.

9. Verwendung nach Anspruch 8, **dadurch gekennzeichnet, dass** das Medikament für Patienten vorgesehen ist, bei denen diese Standardtherapie nicht wirksam oder nicht mehr wirksam ist.

## Revendications

1. Utilisation de peptides polycationiques pour la préparation et la fabrication de médicaments ou de compositions pharmaceutiques, comprenant des antigènes du VHC ou des épitopes du VHC pour le traitement de patients avec des infections à VHC chroniques.

2. Utilisation selon la revendication 1, dans laquelle les médicaments ou les compositions pharmaceutiques sont des vaccins.

3. Utilisation selon la revendication 1 ou 2, **caractérisée en ce que** les patients sont ceux qui n'ont pas répondu, ont partiellement répondu, ou ont rechuté d'une thérapie du VHC standard primaire par une combinaison d'interféron-alpha PEGylé et de l'antiviral ribavirine.

4. Utilisation selon l'une quelconque des revendications 1 à 3, **caractérisée en ce que** les peptides polycationiques contiennent entre 20 et 500 résidus d'acide aminé.

5. Utilisation selon la revendication 4, **caractérisée en ce que** les peptides polycationiques contiennent entre 30 et 200 résidus d'acide aminé.

6. Utilisation selon l'une quelconque des revendications 1 à 5, **caractérisée en ce que** le peptide polycationique est une polyarginine ou une polylysine.

7. Utilisation selon l'une quelconque des revendications 1 à 6, **caractérisée en ce que** le médicament est utilisé pour l'induction de cellules T auxiliaires CD4+ et de cellules T cytotoxiques CD8+.

8. Utilisation selon l'une quelconque des revendications 1 à 7, **caractérisée en ce que** le médicament est utilisé pour remplacer ou complémenter une thérapie standard du VHC avec de l'interféron alpha et de la ribavirine.

9. Utilisation selon la revendication 8, **caractérisée en ce que** le médicament est destiné à des patients où une telle thérapie standard n'est pas efficace ou plus du tout efficace.
